(19) Europäisches Patentamt
European Patent Office
Office européen des brevets

(11) EP 0 735 022 A2

(12) EUROPÄISCHE PATENTANMELDUNG

(43) Veröffentlichungstag:
**02.10.1996 Patentblatt 1996/40**

(51) Int. Cl.⁶: **C07C 303/32**, C07C 309/10

(21) Anmeldenummer: **96104373.4**

(22) Anmeldetag: **20.03.1996**

(84) Benannte Vertragsstaaten:
**AT BE CH DE DK ES FR GB IT LI NL**

(30) Priorität: **29.03.1995 DE 19511461**

(71) Anmelder: **HOECHST AKTIENGESELLSCHAFT**
**65929 Frankfurt am Main (DE)**

(72) Erfinder:
- **Müller, Wolf-Dieter, Dr.**
  **Charlotte, NC 28277 (US)**
- **Bühring, Dirk, Dr.,**
  **Rua Particular casa 35**
  **08613-010 Suzano - SP (BR)**
- **Zerrer, Ralf, Dr.**
  **63755 Alzenau (DE)**

# (54) Verfahren zur Herstellung von fremdsalzfreien alkoxylierten Acyloxyalkansulfonaten

(57) Die hergestellten Acyloxyalkansulfonate entsprechen der Formel 1

$$R\text{-}\overset{\overset{O}{\|}}{C}\text{-}O\text{-}CHR^1\text{-}CHR^2\text{-}O\text{-}CHR^3\text{-}CHR^4\text{-}SO_3X \qquad (1)$$

worin bedeuten: R einen Kohlenwasserstoffrest mit 5 bis 31 Kohlenstoffatomen, X ein Alkalimetall oder Ammonium, $R^1$ und $R^2$, gleich oder verschieden, Wasserstoff, ein $C_1$ bis $C_4$-Alkyl oder ein Hydroxy-$C_1$ bis $C_4$-alkyl, $R^3$ und $R^4$, gleich oder verschieden, Wasserstoff oder ein $C_1$ bis $C_4$-Alkyl. Die fremdsalzfreien Produkte werden in der Weise erhalten, daß man a) eine Glykolverbindung der Formel 2 $HO\text{-}CHR^1\text{-}CHR^2\text{-}OH$ (2) mit einem Hydroxyalkansulfonat der Formel 3 $HO\text{-}CHR^3\text{-}CHR^4\text{-}SO_3X$ (3) in Gegenwart eines alkalischen Katalysators umsetzt zur Bildung eines alkoxylierten Hydroxyalkansulfonates der Formel 4 $HO\text{-}CHR^1\text{-}CHR^2\text{-}O\text{-}CHR^3\text{-}CHR^4\text{-}SO_3X$ (4) und b) das im Schritt a) erhaltene alkoxylierte Hydroxyalkansulfonat mit einer Fettsäure der Formel 5 R-COOH (5) in Gegenwart eines Veresterungskatalysators umsetzt zur Bildung eines alkoxylierten Acyloxyalkansulfonates der angegebenen Formel 1.

EP 0 735 022 A2

**Beschreibung**

Die Erfindung betrifft ein Verfahren zur Herstellung von fremdsalzfreien alkoxylierten Acyloxyalkansulfonaten der nachstehenden Formel 1

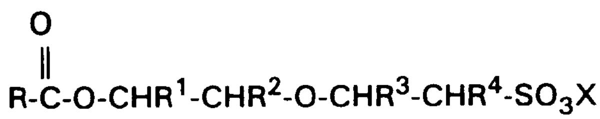
(1)

worin bedeuten:

| | |
|---|---|
| R | einen Kohlenwasserstoffrest mit 5 bis 31 Kohlenstoffatomen, |
| X | ein Alkalimetall oder Ammonium, |
| $R^1$ und $R^2$, | gleich oder verschieden, Wasserstoff, ein $C_1$ bis $C_4$-Alkyl oder ein Hydroxy-$C_1$ bis $C_4$-alkyl, |
| $R^3$ und $R^4$, | gleich oder verschieden, Wasserstoff oder ein $C_1$ bis $C_4$-Alkyl. |

Alkoxylierte Acyloxyalkansulfonate sind wertvolle anionische Tenside, die vor allem zur Bereitung von Syndet-Seifen, kosmetischen Mitteln und Reinigungsformulierungen eingesetzt werden. Sie weisen im Vergleich zu nicht alkoxylierten Acyloxyalkansulfonaten ein höheres Schaumvermögen, höhere Hartwasserstabilität und bessere Hautverträglichkeit auf.

In EP-A-0 585 071 (US-A-5 384 421) wird die Herstellung von nicht alkoxylierten Acyloxyalkansulfonaten durch Veresterung von Fettsäuren mit nicht alkoxylierten Hydroxyalkansulfonaten beschrieben (Direktveresterung). Dabei werden die Fettsäure und das Salz der Hydroxyalkansulfonsäure in Gegenwart eines Veresterungskatalysators und eines Konsistenzreglers bei einer Temperatur von 180 bis 240 °C umgesetzt unter gleichzeitiger Entfernung von vorhandenem Wasser. Als Konsistenzregler werden bestimmte Paraffine eingesetzt. Der Einsatz derartiger Verbindungen ist erforderlich, weil mit fortschreitender Veresterung das Reaktionsgemisch hochviskos wird. Mit Konsistenzreglern wird eine niedrigere Viskosität des Reaktionsgemisches erreicht und die Umsetzung erleichtert, das Veresterungsprodukt enthält aber die eingesetzten Verbindungen, wodurch das angestrebte Acyloxyalkansulfonat mehr oder weniger verdünnt erhalten wird.

In EP-A-0 544 478 werden alkoxylierte Acyloxyalkansulfonate und ein aus zwei Schritten bestehendes Verfahren zu ihrer Herstellung beschrieben. Im ersten Schritt werden alkoxylierte Hydroxyalkansulfonate durch Sulfonierung der entsprechenden Chlorverbindungen mit Natriumbisulfit bereitet, die dann in einem zweiten Schritt mit Fettsäurechlorid oder Fettsäure zu alkoxylierten Acyloxyalkansulfonaten umgesetzt werden. Die folgenden Reaktionsgleichungen mit 2-(2-Chloroethoxy)ethanol und Laurinsäure sollen dies näher veranschaulichen:

$$HO\text{-}CH_2CH_2OCH_2CH_2\text{-}Cl + Na_2SO_3 \rightarrow HO\text{-}CH_2CH_2OCH_2CH_2\text{-}SO_3Na + NaCl$$

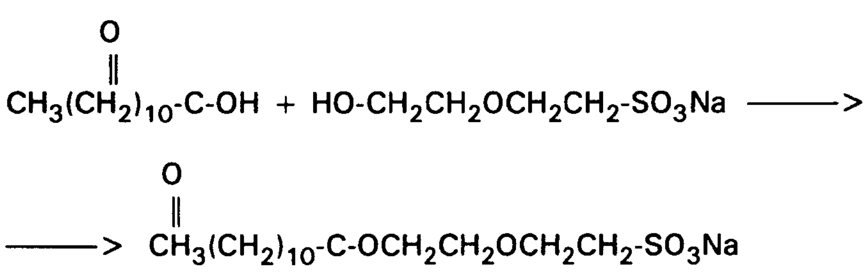

Das im ersten Schritt anfallende Alkalimetallhalogenid NaCl ist auch im Endprodukt (Natriumlauroyldiethoxyisethionat) enthalten. Die Abtrennung des NaCl vom Zwischenprodukt 2-(2-Sulfoethoxy)ethanol könnte nur mit einem hohen technischen Aufwand erreicht werden. Die so hergestellten alkoxylierten Acyloxyalkansulfonate enthalten also

in der Regel eine mehr oder weniger große Menge vom Fremdsalz Alkalimetallhalogenid. Das bekannte Verfahren hat den weiteren Nachteil, daß es zur großtechnischen Herstellung von fremdsalzfreien Produkten aufgrund des hohen Aufwandes zur Fremdsalz-Abtrennung kaum geeignet ist.

Die Aufgabe der Erfindung besteht darin, ein auch großtechnisch durchführbares Verfahren vorzuschlagen, mit dem fremdsalzfreie und damit sehr reine alkoxylierte Acyloxyalkansulfonate erhalten werden.

Das erfindungsgemäße Verfahren ist dadurch gekennzeichnet, daß man

a) eine Glykolverbindung der Formel 2 $HO\text{-}CHR^1\text{-}CHR^2\text{-}OH$ (2), worin $R^1$ und $R^2$ die angegebenen Bedeutungen haben, mit einem Hydroxyalkansulfonat der Formel 3 $HO\text{-}CHR^3\text{-}CHR^4\text{-}SO_3X$ (3), worin $R^3$, $R^4$ und X die angegebenen Bedeutungen haben, in Gegenwart eines alkalischen Katalysators umsetzt zur Bildung eines alkoxylierten Hydroxyalkansulfonates der Formel 4 $HO\text{-}CHR^1\text{-}CHR^2\text{-}O\text{-}CHR^3\text{-}CHR^4\text{-}SO_3X$ (4) und

b) das im Schritt a) erhaltene alkoxylierte Hydroxyalkansulfonat mit einer Fettsäure der Formel 5 R-COOH (5), worin R die angegebene Bedeutung hat, in Gegenwart eines Veresterungskatalysators umsetzt zur Bildung eines alkoxylierten Acyloxyalkansulfonates der angegebenen Formel 1.

$R^1$ und $R^2$ sind vorzugsweise jeweils H oder $R^1$ ist H und $R^2$ $CH_3$, so daß $-CHR^1\text{-}CHR^2-$ den Ethylenrest oder den Isopropylenrest darstellen. $R^3$ und $R^4$ sind vorzugsweise jeweils H. X ist vorzugsweise Natrium, Kalium oder Ammonium. Was die Fettsäure RCOOH betrifft, ist R ein Kohlenwasserstoffrest mit 5 bis 31 Kohlenstoffatomen, der gesättigt oder ungesättigt, gerade oder verzweigt sein kann, wobei gerade (unverzweigt) bevorzugt ist. R kann auch ein Gemisch von solchen Kohlenwasserstoffresten sein. R ist bevorzugt $C_5$ bis $C_{21}$-Alkyl oder $C_5$ bis $C_{21}$-Alkenyl oder eine Mischung davon. Die Alkyl- und Alkenylreste sind vorzugsweise unverzweigt. Die Alkenylreste sind ferner vorzugsweise ein- bis dreifach ungesättigt. Als Beispiele von Fettsäuren seien genannt Capronsäure, Caprinsäure, Laurinsäure, Myristinsäure, Stearinsäure, Arachinsäure, Ölsäure, Linolsäure, Linolensäure, Cocosfettsäure und Talgfettsäure sowie Mischungen davon.

Beim erfindungsgemäßen Verfahren wird zunächst ein alkoxyliertes Hydroxyalkansulfonat in der Weise hergestellt, daß man eine Glykolverbindung der Formel 2, vorzugsweise Ethylenglykol (Ethandiol-1,2) oder Propylenglykol (Propandiol-1,2), mit einer Hydroxyalkansulfonatverbindung der Formel 3, vorzugsweise mit einem Kalium-, Natrium- oder Ammonium-Hydroxyethansulfonat (Isethionat), in Gegenwart eines basischen Katalysators, vorzugsweise NaOH oder KOH, umsetzt. Der erste Schritt des erfindungsgemäßen Verfahrens, das ist eine basekatalysierte Kondensationsreaktion, wird bei einer Temperatur von im allgemeinen 150 bis 250 °C, vorzugsweise 180 bis 210 °C, und unter Normaldruck durchgeführt, wobei anwesendes Wasser (das ist das in die Reaktionsmischung gegebenenfalls eingebrachte und das bei der Reaktion entstandene Wasser) entfernt wird, vorzugsweise durch kontinuierliches Abdestillieren. Die Glykolverbindung der Formel 2, das Hydroxyalkansulfonat der Formel 3 und die basekatalysierende Verbindung werden im allgemeinen im Molverhältnis von 2 bis 7 : 1 : 0,1 bis 0,2 eingesetzt, vorzugsweise im Molverhältnis von 2 bis 5 : 1 : 0,1 bis 0,2. Die Reaktionszeit liegt bei etwa 2 bis 10 Stunden. Das mehr oder weniger stark alkalische Reaktionsprodukt mit der im Überschuß eingesetzten Glykolverbindung wird vorzugsweise derart weiterbehandelt, daß im Schritt a) ein Produkt mit einem hohen Gehalt an alkoxyliertem Hydroxyalkansulfonat und ohne störende Anteile für die nachfolgende Veresterungsreaktion erhalten wird. Diese Behandlung führt man bevorzugt wie folgt durch: Das alkalische Reaktionsprodukt wird zunächst mit einer solchen Hydroxyalkansulfonsäure neutralisiert, die dem mit der Kondensationsreaktion hergestellten Hydroxyalkansulfonat entspricht. Hat man also durch Umsetzung von zum Beispiel Ethylenglykol ($HOCH_2CH_2OH$) und Natriumisethionat ($HOCH_2CH_2SO_3Na$) in Gegenwart von Natriumhydroxid die Verbindung $HO\text{-}CH_2CH_2OCH_2CH_2\text{-}SO_3Na$ hergestellt, wird man dieses Reaktionsprodukt mit $HO\text{-}CH_2CH_2OCH_2CH_2\text{-}SO_3H$ neutralisieren. Vom neutralisierten Reaktionsprodukt wird dann die im Überschuß eingesetzte Glykolverbindung abgetrennt, zum Beispiel mit Hilfe eines Dünnschichtverdampfers, wobei man je nach Glykolverbindung eine Temperatur von etwa 120 bis 250 °C und einen Druck von etwa 10 bis 500 mbar einhält. Das so erhaltene Produkt besteht im wesentlichen aus einem oder mehreren alkoxylierten Hydroxyalkansulfonaten.

Im Schritt b) des erfindungsgemäßen Verfahrens wird das im Schritt a) erhaltene Produkt mit einer oder mehreren Fettsäuren in Gegenwart eines Katalysators umgesetzt. Geeignete Veresterungskatalysatoren sind in der genannten EP-A-0 585 071 ausführlich beschrieben, die hier miteinbezogen wird. Es handelt sich um Alkansulfonsäuren, Hydroxyalkansulfonsäuren, Arylsulfonsäuren, anorganischen Säuren wie Schwefelsäure, Phosphorsäure, phosphorige Säure, Borsäure oder deren Anhydride, Schwermetallsalze wie Zinksulfat, Zirkoniumsulfat, Zinkisethionat, Zinkborat, Aluminiumsulfat, Titansulfat oder Wolframphosphat, Metalloxide wie Zinkoxid, Aluminiumoxid, Magnesiumoxid, Ceroxid, Zirkoniumoxid oder Lanthanoxid, ferner um Mischungen von zwei oder mehreren der genannten Katalysatoren und um Seifen, die aus Schwermetallen und Metalloxiden gebildet werden. Ein besonders bevorzugter Veresterungskatalysator ist Zinkoxid. Der Veresterungskatalysator wird in einer Menge von im allgemeinen 0,05 bis 2 Gew.-%, vorzugsweise 0,05 bis 1 Gew.-%, eingesetzt, Gewichtsprozente bezogen auf Fettsäure und alkoxyliertes Hydroxyalkansulfonsäuresalz.

Die Umsetzung von Fettsäure und alkoxyliertem Hydroxyalkansulfonsäuresalz, im allgemeinen im Molverhältnis von 1 : 1 bis 2 : 1, vorzugsweise etwa 1 : 1, wird erfindungsgemäß bei einer Temperatur von 180 bis 250 °C, vorzugsweise 200 bis 230 °C, durchgeführt. Das gegebenenfalls mit den Ausgangskomponenten in die Reaktionsmischung eingebrachte Wasser und das durch die Veresterungsreaktion entstehende Wasser wird kontinuierlich aus der Reaktionsmischung ausgetragen. Die Reaktionsmischung ist bis zum Ende der Umsetzung, auch bei 100%igem Umsatz, homogen, relativ niedrigviskos und gut rührbar, so daß Konsistenzregler zur weiteren Herabsetzung der Viskosität im allgemeinen nicht erforderlich sind. Geeignete Produkte dafür wären zum Beispiel Paraffine und Fettsäureester in einer Menge von etwa 5 bis 20 Gew.-%, bezogen auf die gesamte Reaktionsmischung. Die Zeit bis zum angestrebten Umsatz von Fettsäure oder von alkoxyliertem Hydroxyalkansulfonat liegt bei etwa 4 bis 8 Stunden. Im allgemeinen wird man, zum Beispiel aus Zeitgründen, 100%igen Umsatz nicht anstreben, sondern bei einem niedrigeren Prozentsatz, zum Beispiel bei 70 bis 90 Gew.-% alkoxyliertem Acyloxyalkansulfonat, die Veresterungsreaktion abbrechen.

Der erfindungsgemäße Veresterungsschritt kann im einzelnen zum Beispiel in der Weise durchgeführt werden, daß man - bei Atmosphärendruck - die Fettsäure, das Salz der alkoxylierten Hydroxyalkansulfonsäure und den Veresterungskatalysator in einem Reaktionsgefäß vorlegt und die Mischung unter Rühren auf die angegebene Temperatur erhitzt. Vorhandenes Wasser destilliert bereits während des Erhitzens der Reaktionsmischung und dann weiter während der Veresterungsreaktion kontinuierlich ab. Die Veresterung kann auch nach der in EP-A-0 585 071 beschriebenen Methode durchgeführt werden. Hier wird die Reaktion teils bei Atmosphärendruck und teils unter Anwendung eines Vakuums zur schnelleren Austragung des Wassers durchgeführt. Nach Erreichen des angestrebten Umsetzungsgrades wird die Veresterungsreaktion zum Beispiel durch Abkühlen beendet. Das erhaltene Reaktionsprodukt ist bei Raumtemperatur flüssig oder fest. Eine Konfektionierung des bei Raumtemperatur festen Produktes kann man zum Beispiel mit Hilfe einer Schuppenwalze oder eines Kühlbandes durchführen.

Mit dem erfindungsgemäßen Verfahren, das auch großtechnisch durchführbar ist, können fremdsalzfreie alkoxylierte Acyloxyalkansulfonate mit hoher Reinheit hergestellt werden. Die Produkte weisen einen hohen Gehalt an alkoxyliertem Acyloxyalkansulfonat auf und als weitere vorteilhafte Eigenschaften gute Wasserlöslichkeit, starke Schaumbildung und gute Hartwasserstabilität sowie ein gut wirkendes Hautgefühl. Die erfindungsgemäß erhaltenen Produkte eignen sich deshalb vor allem auch für wäßrige Formulierungen. Durch die Direktveresterung (Direktkondensation) kann auf die Verwendung von Fettsäurechloriden verzichtet werden, die sonst in einem gesonderten Schritt aus Fettsäure hergestellt werden müßten. Die Verwendung von Kosistenzreglern und/oder Verdünnungsmitteln, die in der Regel keine Wertstoffe für zum Beispiel Reinigungsformulierungen und kosmetischen Mitteln darstellen, ist nicht notwendig. Das feste oder flüssige Reaktionsprodukt enthält, wie oben erwähnt, im allgemeinen 70 bis 90 Gew.-% alkoxyliertes Acyloxyalkansulfonat der angegebenen Formel 1, Gewichtsprozente bezogen auf das feste oder flüssige Gesamtprodukt.

Die Erfindung wird nun an Beispielen noch näher erläutert. Prozentangaben beziehen sich auf das Gewicht, wenn keine anderen Angaben gemacht sind.

Beispiel 1

a) Herstellung von einem alkoxylierten Hydroxyalkansulfonat:

In einem 1-l-Planschliffbecher mit Ankerrührer, absteigender Destillationsbrücke und Innenthermometer werden 310 g (5 mol) Ethandiol-1,2, 148 g (1 mol) 2-Hydroxyethansulfonsäure-Natriumsalz (Natriumisethionat) und 4 g (0,1 mol) Natriumhydroxid vorgelegt. Das Reaktionsgemisch wird auf eine Temperatur von 190 bis 195 °C erhitzt. Innerhalb von 3 Stunden destillieren circa 18 g Wasser aus dem Reaktionsgemisch ab. Nach dem Abkühlen auf Raumtemperatur wird die alkalische Produktlösung durch Versetzen mit circa 17 g (0,1 mol) 2-(2-Hydroxyethoxy)ethansulfonsäure neutralisiert. Aus der neutralen glykolhaltigen Produktlösung wird das Ethersulfonat mittels einer Dünnschichtverdampfung gewonnen. (Die Mantelheizung des Verdampferrohres wird bei etwa 200 °C und das Vakuum bei etwa 10 mbar gehalten.) Das 2-(2-Hydroxyethoxy)ethansulfonsäure-Natriumsalz (Diglykolsulfonsäure-Natriumsalz) fällt als farbloser Destillationsrückstand mit einem Schmelzbereich von 140 bis 150 °C an. Der Reinheitsgrad des Produktes beträgt 98 %, der Glykolgehalt ist < 1 %.

b) Veresterung eines alkoxylierten Hydroxyalkansulfonates mit Fettsäure zum alkoxylierten Acyloxyalkansulfonat:

In einem 1-l-Planschliffbecher mit Ankerrührer, absteigender Destillationsbrücke, Innenthermometer und Stickstoffeinleitung werden 285 g (1 mol) Stearinsäure, 196 g (1 mol) von dem oben beschriebenen Natrium-2-(2-hydroxyethoxy)ethansulfonat und 0,5 g Zinkoxid vorgelegt. Der Ansatz wird auf 200 °C erwärmt und das bei der Direktkondensation gebildete Wasser abdestilliert. Bei einem Natrium-2-(2-stearoyloxyethoxy)ethansulfonat-Gehalt von 70 % wird die Reaktion abgebrochen und das Produkt zum Erkalten auf ein Blech gegossen. Das Endprodukt besteht im wesentlichen aus 70 % Natrium-2-(2-stearoyloxyethoxy)ethansulfonat, 17 % Stearinsäure und 13 % Natrium-2-(2-hydroxyethoxy)ethansulfonat.

Das oben beschriebene Natrium-2-(2-hydroxyethoxy)ethan-sulfonat wird auch in den weiteren Beispielen eingesetzt.

Beispiel 2

In der Einrichtung vom Beispiel 1 werden 200 g (1 mol) Laurinsäure, 196 g (1 mol) Natrium-2-(2-hydroxyethoxy)ethan-sulfonat und 0,5 g Zinkoxid vorgelegt. Die Mischung wird auf 200 °C erwärmt und das bei der Direktkondensation gebildete Wasser abdestilliert. Bei einem Wirkstoffgehalt von 79 % wird die Reaktion abgebrochen und das Produkt zum Erkalten auf ein Blech gegossen. Das Reaktionsprodukt besteht im wesentlichen aus 79 % Natrium-2-(2-lauroyloxyethoxy)ethan-sulfonat (Wirkstoff), 9 % Laurinsäure und 12 % Natrium-2-(2-hydroxyethoxy)ethansulfonat.

Beispiel 3

In der Einrichtung vom Beispiel 1 werden 158 g (0,8 mol) Cocosfettsäure mit einem mittleren Molekulargewicht von 205, 158 g (0,8 mol) Natrium-2-(2-hydroxyethoxy)ethansulfonat und 0,5 g Zinkoxid vorgelegt. Der Ansatz wird auf 200 °C erwärmt und das bei der Direktkondensation gebildete Wasser abdestilliert. Bei einem Wirkstoffgehalt von 80 % wird die Reaktion abgebrochen und das Produkt zum Erkalten auf ein Blech gegossen. Das Endprodukt besteht im wesentlichen aus 80 % Natrium-2-(2-cocoyloxyethoxy)ethansulfonat, 9 % Cocosfettsäure und 11 % Natrium-2-(2-hydroxyethoxy)ethan-sulfonat.

Beispiel 4

In der Einrichtung vom Beispiel 1 werden 289 g (1,3 mol) Cocosfettsäure mit einem mittleren Molekulargewicht von 218, 196 g (1 mol) Natrium-2-(2-hydroxyethoxy)ethansulfonat und 0,8 g Zinkoxid vorgelegt. Der Mischung wird auf 220 °C erwärmt und das bei der Direktkondensation gebildete Wasser abdestilliert. Bei einem Wirkstoffgehalt von 75 % wird die Reaktion abgebrochen und das Produkt zum Erkalten auf ein Blech gegossen. Es besteht im wesentlichen aus 75 % Natrium-2-(2-cocoyloxyethoxy)ethansulfonat, 20 % Cocosfettsäure und 5 % Natrium-2-(2-hydroxyethoxy)ethansulfonat.

Beispiel 5

In einem 360-l-Rührkessel mit Ankerrührer, absteigender Destillationsvorrichtung, Stickstoffeinleitung und Temperaturmessung im Kessel werden 43 kg (224 mol) Natrium-2-(2-hydroxyethoxy)ethansulfonat, 50 kg (250 mol) Laurinsäure und 0,2 kg Zinkoxidpulver vorgelegt und anschließend unter Destillation von Wasser auf 200 °C erhitzt. Nach Erreichen eines Wirkstoffgehaltes von 73 % wird das Reaktionprodukt auf 130 °C abgekühlt und über eine Schuppenwalze konfektioniert. Das geschuppte Produkt besteht im wesentlichen aus 73 % Natrium-2-(2-lauroyloxyethoxy)ethansulfonat, 17 % Laurinsäure und 10 % Natrium-2-(2-hydroxyethoxy)ethansulfonat.

## Patentansprüche

1. Verfahren zur Herstellung von fremdsalzfreien alkoxylierten Acyloxyalkansulfonaten der nachstehenden Formel 1

$$R-\overset{\overset{\displaystyle O}{\|}}{C}-O-CHR^1-CHR^2-O-CHR^3-CHR^4-SO_3X \qquad (1)$$

worin bedeuten:

| | |
|---|---|
| R | einen Kohlenwasserstoffrest mit 5 bis 31 Kohlenstoffatomen, |
| X | ein Alkalimetall oder Ammonium, |
| $R^1$ und $R^2$, | gleich oder verschieden, Wasserstoff, ein $C_1$ bis $C_4$-Alkyl oder ein Hydroxy-$C_1$ bis $C_4$-alkyl, |
| $R^3$ und $R^4$, | gleich oder verschieden, Wasserstoff oder ein $C_1$ bis $C_4$-Alkyl, |

dadurch gekennzeichnet, daß man

a) eine Glykolverbindung der Formel 2 $HO-CHR^1-CHR^2-OH$ (2), worin $R^1$ und $R^2$ die angegebenen Bedeutungen haben, mit einem Hydroxyalkansulfonat der Formel 3 $HO-CHR^3-CHR^4-SO_3X$ (3), worin $R^3$, $R^4$ und X die angegebenen Bedeutungen haben, in Gegenwart eines alkalischen Katalysators umsetzt zur Bildung eines alkoxylierten Hydroxyalkansulfonates der Formel 4 $HO-CHR^1-CHR^2-O-CHR^3-CHR^4-SO_3X$ (4) und

b) das im Schritt a) erhaltene alkoxylierte Hydroxyalkansulfonat mit einer Fettsäure der Formel 5 R-COOH (5), worin R die angegebene Bedeutung hat, in Gegenwart eines Veresterungskatalysators umsetzt zur Bildung eines alkoxylierten Acyloxyalkansulfonates der angegebenen Formel 1.

**2.** Verfahren nach Anspruch 1, dadurch gekennzeichnet, daß R einen unverzweigten $C_5$ bis $C_{21}$-Alkylrest, einen unverzweigten $C_5$ bis $C_{21}$-Alkenylrest oder eine Mischung davon, $R^1$ und $R^2$ jeweils H oder $R^1$ H und $R^2$ $CH_3$ und $R^3$ und $R^4$ jeweils H und X ein Alkalimetall oder Ammonium bedeuten.

**3.** Verfahren nach Anspruch 1 oder 2, dadurch gekennzeichnet, daß im Schritt a) die Glykolverbindung, das Hydroxyalkansulfonat und Natriumhydroxid oder Kaliumhydroxid als Katalysator im Molverhältnis von 2 bis 7 : 1 : 0,1 bis 0,2 eingesetzt werden und die Umsetzung bei einer Temperatur von 150 bis 250 °C unter Entfernung des anwesenden Wassers durchgeführt wird.

**4.** Verfahren nach Anspruch 1 oder 2, dadurch gekennzeichnet, daß der Schritt a) in der Weise durchgeführt wird, daß man

$a_1$) die Glykolverbindung, das Hydroxyalkansulfonat und Natrium- oder Kaliumhydroxid als Katalysator im Molverhältnis von 2 bis 5 : 1 : 0,1 bis 0,2 einsetzt und die Umsetzung bei einer Temperatur von 180 bis 210 °C unter Entfernung des anwesenden Wassers durchführt,
$a_2$) das im Schritt $a_1$) erhaltene alkalische Reaktionsprodukt mit einer solchen Hydroxyalkansulfonsäure neutralisiert, die dem im Schritt $a_1$) gebildeten alkoxylierten Hydroxyalkansulfonat entspricht, und
$a_3$) die im neutralisierten Reaktionsprodukt anwesende Glykolverbindung abtrennt, wobei ein Produkt aus im wesentlichen alkoxyliertem Hydroxyalkansulfonat erhalten wird.

**5.** Verfahren nach einem oder mehreren der Ansprüche 1 bis 4, dadurch gekennzeichnet, daß im Schritt b) die Fettsäure und das alkoxylierte Hydroxyalkansulfonat im Molverhältnis von 1 : 1 bis 2 : 1 eingesetzt werden und die Umsetzung bei einer Temperatur von 180 bis 250 °C unter Entfernung des anwesenden Wassers durchgeführt wird.

**6.** Verfahren nach einem oder mehreren der Ansprüche 1 bis 4, dadurch gekennzeichnet, daß im Schritt b) die Fettsäure und das alkoxylierte Hydroxyalkansulfonat im Molverhältnis von etwa 1 : 1 eingesetzt werden und die Umsetzung bei einer Temperatur von 200 bis 230 °C unter Entfernung des anwesenden Wassers durchgeführt wird.

**7.** Verfahren nach einem oder mehreren der Ansprüche 1 bis 6, dadurch gekennzeichnet, daß als Veresterungskatalysator Zinkoxid in einer Menge von 0,05 bis 2 Gew.-%, bezogen auf Fettsäure und alkoxyliertes Hydroxyalkansulfonat, eingesetzt wird.

**8.** Verfahren nach Anspruch 1, dadurch gekennzeichnet, daß man

$a_1$) eine Glykolverbindung der Formel 2 $HO-CHR^1-CHR^2-OH$ (2), worin $R^1$ und $R^2$ die angegebenen Bedeutungen haben, ein Hydroxyalkansulfonat der Formel 3 $HO-CHR^3-CHR^4-SO_3X$ (3), worin $R^3$, $R^4$ und X die angegebenen Bedeutungen haben, und Natrium- oder Kaliumhydroxid als Katalysator im Molverhältnis von 2 bis 7 : 1 : 0,1 bis 0,2 einsetzt und die Umsetzung bei einer Temperatur von 150 bis 250 °C unter Entfernung des anwesenden Wassers durchführt,
$a_2$) das im Schritt $a_1$) erhaltene alkalische Reaktionsprodukt mit einer solchen Hydroxyalkansulfonsäure neutralisiert, die dem im Schritt $a_1$) gebildeten alkoxylierten Hydroxyalkansulfonat entspricht, und
$a_3$) die im neutralisierten Reaktionsprodukt anwesende Glykolverbindung abtrennt, wobei ein Produkt aus im wesentlichen alkoxyliertem Hydroxyalkansulfonat der Formel 4 $HO-CHR^1-CHR^2-O-CHR^3-CHR^4-SO_3X$ (4) erhalten wird, und
b) das in den Schritten $a_1$) bis $a_3$) erhaltene alkoxylierte Hydroxyalkansulfonat mit einer Fettsäure der Formel 5 R-COOH (5), worin R die angegebene Bedeutung hat, im Molverhältnis von Fettsäure zu alkoxyliertem Hydroxyalkansulfonat von 1 : 1 bis 2 : 1 in Gegenwart von Zinkoxid in einer Menge von 0,05 bis 2 Gew.-%, bezogen auf Fettsäure und alkoxyliertem Hydroxyalkansulfonat, bei einer Temperatur von 180 bis 250 °C unter Entfernung des anwesenden Wassers umsetzt zur Bildung des angestrebten alkoxylierten Acyloxyalkansulfonates.

**9.** Verfahren nach Anspruch 8, dadurch gekennzeichnet, daß im Schritt $a_1$) die Glykolverbindung, das Hydroxyalkansulfonat und das Natrium- oder Kaliumhydroxid im Molverhältnis von 2 bis 5 : 1 : 0,1 bis 0,2 eingesetzt werden und

die Umsetzung bei einer Temperatur von 180 bis 210 °C durchgeführt wird und daß im Schritt b) bei einer Temperatur von 200 bis 230 °C umgesetzt wird.

**10.** Verfahren nach Anspruch 8 oder 9, dadurch gekennzeichnet, daß R einen unverzweigten $C_5$ bis $C_{21}$-Alkylrest, einen unverzweigten $C_5$ bis $C_{21}$-Alkenylrest oder eine Mischung davon, $R^1$ und $R^2$ jeweils H oder $R^1$ H und $R^2$ $CH_3$ und $R^3$ und $R^4$ jeweils H und X ein Alkalimetall oder Ammonium bedeuten.